Europäisches Patentamt

European Patent Office

Office européen des brevets

(19)

(11) Numéro de publication: **0 000 464**
**B1**

(12) **FASCICULE DE BREVET EUROPEEN**

(45) Date de publication du fascicule de brevet: **12.08.81**

(51) Int. Cl.³: **C 07 D 277/72**

(21) Numéro de dépôt: **78400052.3**

(22) Date de dépôt: **03.07.78**

(54) Procédé de purification du mercaptobenzothiazole.

(30) Priorité: **12.07.77 FR 7721437**

(43) Date de publication de la demande:
**24.01.79 Bulletin 79/2**

(45) Mention de la délivrance du brevet:
**12.08.81 Bulletin 81/32**

(84) Etats Contractants Désignés:
**BE CH DE FR GB LU NL**

(56) Documents cités:
**DE - A - 2 258 484**
**US - A - 3 030 373**

**CHEMICAL ABSTRACTS, vol. 65 (1966) 2268f &
SU - A - 179 306 (SCIENTIFIC RESEARCH
INSTITUTE OF ORGANIC INTERMEDIATES AND
DYES AND ANILINE DYE PLANT, BEREZNIKI)**

(73) Titulaire: **P C U K PRODUITS CHIMIQUES UGINE
KUHLMANN**
**Service Propriété Industrielle Tour Manhattan**
**F-92087 PARIS LA DEFENSE 2 Cédex 21 (FR)**

(72) Inventeur: **Alicot, Michel Jean Camille**
**La Barthe de Neste**
**F-65300 Lannemezan (FR)**
Inventeur: **Rhode, René**
**379 Quartier Guerissa**
**65300 Lannemezan (FR)**
Inventeur: **Tignel, Adrien, Paul, Noäl**
**15 rue des Pyrénées**
**31210 Montrejeau (FR)**

(74) Mandataire: **Houssin, Jean Produits Chimiques
Ugine Kuhlmann et al,**
**Service Propriété Industrielle Tour Manhattan**
**Cedex 21**
**F-92087 Paris la Defense (FR)**

Courier Press, Leamington Spa, England.

## Procédé de purification du mercaptobenzothiazole

Le mercaptobenzothiazole est connu dans l'industrie de transformation des élastomères pour son utilisation comme accélérateur de vulcanisation. Il est également une matière première importante dans la synthèse d'accélérateurs de vulcanisation plus perfectionnés adaptés aux problèmes particuliers que posent la fabrication d'articles aussi différents que par exemple, les pneumatiques, les câbles électriques, les semelles de chaussures, les joints d'isolation. Il entre aussi pour une large part dans la synthèse de composés phytosanitaires.

La plupart des procédés de fabrication connus font appel à la réaction, en proportions appropriées, à haute température et haute pression, de l'aniline, du soufre et du sulfure de carbone. D'autres font appel, soit à la réaction de la thiocarbanilide, du sulfure de carbone et du soufre (US—A—1.712.968 du 14.5.1929); soit à la réaction de l'orthochlornitrobenzène, de l'hydrogéné sulfuré ou d'un sulfure alcalin et du sulfure de carbone (US—A—1.960.205 du 22.5.1934; PL—A—86988 du 15.12.1976); soit encore à la réaction du benzothiazole et du soufre (DE—A—2.551.060 du 26.5.1976). Le produit de réaction obtenu dans de telles conditions n'est jamais directement utilisable tel quel. Il contient, en effet, des matières premières n'ayant pas réagi, par exemple de l'aniline, des sous-produits et intermédiaires tels que le benzothiazole, l'anilinobenzothiazole. Une purification soigneuse du produit brut de réaction est nécessaire.

De nombreux procédés de purification ont été proposés à ce jour. Ils font appel fondamentalement à deux techniques qui diffèrent principalement par les concentrations, l'ordre d'emploi, la nature des réactifs préconisés, les températures de traitement.

Le schéma de principe de la première technique est le suivant:
—solubilisation du produit de réaction en milieu alcalin (hydroxyde d'ammonium, hydroxyde de sodium chaux), précédé ou non d'un traitement en milieu acide minéral;
—séparation des impuretés insolubles par filtration;
—séparation des impuretés solubles après leur insolubilisation par oxydation et/ou extraction à l'aide d'un solvant;
—précipitation du mercaptobenzothiazole par action d'un acide minéral.

Les brevets US 1.631.871, 2.658.864, 2.730.528 et 3.818.025 et le brevet français 2.135.807 illustrent l'application en totalité ou en partie d'un tel processus.

Dans l'autre technique, les impuretés sont extraites par traitement du produit de réaction par le sulfure de carbone ou une émulsion de sulfure de carbone et d'eau.

Le brevets US 2.090.233, 3.030.373 et 3.031.073 illustrent ce mode de réalisation.

Bien qu'ils soient actuellement exploités industriellement, ces procédés ne sont pas satisfaisants et présentent chacun, en totalité ou en partie, les inconvénients suivants:
—récupération difficile des matières premières n'ayant pas réagi et que l'on a le plus grand intérêt économique à recycler (aniline tout particulièrement),
—nécessité d'opérer à des concentrations peu élevées pour favoriser la précipitation des impuretés, avec pour conséquence des appareillages de grandes dimensions,
—pertes, par dégradation chimique, du mercaptobenzothiazole au cours des réactions d'oxydation destinées à insolubiliser les impuretés solubles en milieu alcalin,
—pertes, par solubilisation dans le sulfure de carbone, du mercaptobenzothiazole ou recyclage inévitable d'une partie des impuretés si la quantité de sulfure de carbone est limitée,
—enfin, et c'est peut-être l'inconvénient majeur de ces procédés, l'obligation d'avoir à traiter avant leur rejet de grands volumes d'effluents aqueux contenant de fortes charges polluantes. Ces traitements sont difficiles et coûteux. Les processus d'oxydation généralement appliqués ne conduisent pas aux molécules simples souhaitées d'azote, de gaz carbonique et d'anhydride sulfureux mais à un taux non acceptable de molécules solubles qui ne sont dégradées que par un traitement biologique complémentaire aggravant lourdement le coût des unités de production.

Or, il a été trouvé, dans les services de la demanderesse, que l'on peut, à partir du produit brut résultant des procédés connus, obtenir du mercaptobenzothiazole avec un haut rendement et dans un grand état de pureté, au moyen d'une technologie simplifiée. Grâce à ce procédé les produits intermédiaires et matières premières n'ayant pas réagi sont aisément recyclables, les sous-produits peuvent être séparés sans difficulté. D'autre part, en raison de l'absence d'utilisation d'eau, les problèmes de traitement d'effluents sont supprimés.

Le procédé selon l'invention pour la purification du mercaptobenzothiazole est caractérisé en ce que le produit brut obtenu suivant les procédés connus est traite en l'absence d'eau par le tétrachlorure de carbone ou le tétrachloro-1,1,2,2 éthylène ($Cl_2 C = C Cl_2$) avec ou sans distillation préalable des produits volatils.

Lorsque le procédé de purification est appliqué sur le produit brut séparé des produits volatils (tels que l'aniline et le benzothiazole, recyclables après récupération), dans une première phase la séparation des produits volatils peut être réalisée par une distillation dans un large intervalle de température (100 à 300°C).

En raison des risques de décomposition thermique du mercaptobenzothiazole, il est préférable que cette température soit inférieure à 220°C, l'intervalle de choix étant de 100 à 150°C. On utilise avantageusement un évaporateur racleur à film mince qui permet de récupérer le produit solide sous forme pulvérulente. Le vide qu'il est nécessaire de maintenir dans l'appareillage est fonction de la température choisie: il est généralement compris entre 1333 et 2,6664 pascal.

Dans une deuxième phase, le mercaptobenzothiazole brut à purifier, obtenu après distillation, est mis en suspension dans le tétrachlorure de carbone ou le tétrachloro-1,1,2,2 éthylène.

Lorsque le procédé de purification est appliqué sans séparation préalable des produits volatils, le produit brut de réaction est directement mis en suspension dans le tétrachlorure de carbone ou le tétrachloro-1,1,2,2 éthylène. La récupération des produits recyclables est effectuée dans ce cas par distillation de la phase solvant obtenue après séparation du mercaptobenzothiazole purifié insolubilisé.

Dans tous les cas, pour des raisons d'économie, on préfère limiter la concentration du produit solide en suspension dans le solvant à une valeur comprise entre 200 et 400 grammes par litre. La dissolution des impuretés est d'autant plus facile que le produit à purifier est plus finement divisé. Tout moyen connu permet de réaliser aisément cette condition.

Le traitement selon l'invention peut être effectué dans un large intervalle de températures:
—de 0°C à 77°C, température d'ébullition du tétrachlorure de carbone à la pression de 101,325 kPa, préférentiellement dans l'intervalle de 10°C à 50°C,
—de 0°C à 121°C, température d'ébullition du tétrachloro-1,1,2,2 éthylène à la pression de 101,325 kPa, préférentiellement dans l'intervalle de 10°C à 80°C.

Effectuer le traitement à des températures inférieures ou supérieures aux intervalles préférentiels précités ne nuit pas à l'invention, mais est sans intérêt particulier et peut compliquer inutilement l'appareillage.

La récupération finale du mercaptobenzothiazole est faite par tout moyen connu de filtration et séchage, sans traitement alcalin préalable. La solution issue de la filtration est soumise à une distillation de manière à récupérer le tétrachlorure ou le tétrachloro-1,1,2,2-éthylène et à séparer les produits à recycler en totalité ou en partie.

Les exemples suivants illustrent l'invention sans la limiter.

Exemple 1.

On prélève, à la température de 180°C, à la sortie d'un réacteur de synthèse de mercaptobenzothiazole et après dégazage de l'hydrogène sulfuré, 1 000 grammes de produit de réaction de l'aniline, du soufre et du sulfure de carbone, dont la composition est la suivante:

— mercaptobenzothiazole      83,5%

— aniline      3,2%

—benzothiazole      2 %

—sous-produits divers      11,3%.

Ce produit est introduit dans un évaporateur racleur dans lequel on établit une température de 130°C et un vide de 2 kPa.

Les matières volatiles distillent et par condensation on recueille 54 grammes d'une huile jaune clair titrant 57,3% en aniline et 35,2% en benzothiazole.

Le produit solide, extrait de l'évaporateur, broyé, est mis en suspension dans 2 300 ml de tétrachlorure de carbone, à la température de 25°C. Après une heure d'agitation, on filtre la suspension, lave avec deux fois 300 ml de tétrachlorure de carbone, essore et sèche.

On obtient 836,5 grammes d'un produit de couleur jaune, clair, titrant 99% en mercaptobenzothiazole et de point de fusion non corrigé 177—180°C. Le rendement de la purification est de 99,2%.

La fraction contenant l'aniline et le benzothiazole est recyclable dans le réacteur de synthèse sans autre traitement. La solution de tétrachlorure de carbone contenant les sousproduits est distillée de manière à récupérer le solvant recyclable. Une fraction non distillable d'un poids de 109 grammes est recueillie; elle peut être recyclée dans la fabrication du mercaptobenzothiazole.

Le mercaptobenzothiazole soumis à purification est indifféremment le produit brut obtenu suivant l'un quelconque des procédés connus pour sa préparation; on peut citer entre autre celui du brevet US 1.631.871.

Exemple 2.

On opère comme à l'exemple 1 mais en utilisant le tétrachloro-1,1,2,2 éthylène. Les résultats sont identiques. Le mercaptobenzothiazole soumis à purification est indifféremment le produit brut obtenu suivant l'un quelconque des procédés connus pour sa préparation.

Exemple 3.

On prélève à 200°C, à la sortie d'un réacteur de synthèse du mercaptobenzothiazole, 1000 grammes de produit de réaction de l'aniline, du soufre et du sulfure de carbone, dont la composition est la suivante:

— mercaptobenzothiazole      83,5%

— aniline      3,2%

— benzothiazole      2 %

— sous-produits divers      11,3%

Ce produit est introduit sous agitation en 30 minutes environ dans 2000 ml de tétrachloro-1,1,2,2 éthylène maintenu par refroidissement à la température de 20 à 25°C.

Après une heure d'agitation, on filtre la suspension de mercaptobenzothiazole, lave avec 2 à 3 fois 300 ml de tétrachloro-1,1,2,2 éthylène, essore et sèche. On obtient 827 grammes de produit de couleur jaune clair, titrant 98,5% en mercaptobenzothiazole de point de fusion non corrigé 177—180°C. Le rendement de purification est de 97,5%.

A partir de la phase solvant récupérée à la filtration, on obtient par distillation 50 grammes d'un mélange d'aniline et de benzothiazole recyclables.

Exemple 4.

On opère comme à l'exemple 3 mais en remplaçant le tétrachloro-1,1,2,2 éthylène par le tétrachlorure de carbone. Les résultats sont identiques.

Avantages Comparatifs

Le procédé selon l'invention, tel qu'illustré par l'exemple 3 donné ci-dessus, est comparé au procédé du brevet américain 3 030 373 précédemment cité. A cet effet le perchlor-éthylène est utilisé comme solvant dans le procédé de l'exemple 3. En ce qui concerne le brevet américain, le disulfure de carbone, le per-chloréthylène et le benzène sont utilisés comme solvants suivant le procédé décrit à l'exemple 1 de ce brevet.

Le procédé selon l'invention, qui exclut la présence d'eau, permet d'obtenir le mercaptobenzothiazole avec un titre de 98,5%, supérieur à ceux obtenus suivant le procédé antérieur, soit 94,6%, 94,6% et 95,3% avec les solvants précités respectivement.

D'autre part, eu égard au procédé du brevet ouest-allemand 2 258 484, le procédé selon l'invention présente l'avantage d'être plus simple quant au nombre des traitements ou manipulations (en particulier, la préparation du mercaptobenzothiazole sous forme de sel de sodium n'est pas nécessaire) et quant aux installations technologiques.

**Revendications**

1. Procédé pour la purification du mercaptobenzothiazole préparé suivant les procédés connus, caractérisé en ce que le produit brut est traité en l'absence d'eau par le tétrachlorure de carbone ou le tétrachloro-1,1,2,2 éthylène.

2. Procédé tel que revendiqué selon la revendication 1 dans lequel le produit brut est traité par le tétrachlorure de carbone ou le tétrachloro-1,1,2,2 éthylène sans séparation préalable des matières volatiles.

3. Procédé tel que revendiqué selon la revendication 1 dans lequel le produit brut est traité par le tétrachlorure de carbone ou le tétrachloro-1,1,2,2 éthylène après séparation préalable des matières volatiles par distillation sous pression réduite.

4. Procédé tel que revendiqué selon la revendication 3 dans lequel la distillation est effectuée à une température inférieure à 220°C sous une pression de 1333 à 26664 pascal.

5. Procédé tel que revendiqué selon chacune des revendications 1—3 caractérisé en ce que le produit brut est mis en suspension dans le tétrachlorure de carbone ou le tétrachloro-1,1,2,2 éthylène à une température égale ou inférieure à la température d'ébullition du solvant.

6. Procédé tel que revendiqué selon chacune des revendications 1—5 caractérisé en ce que le mercaptobenzothiazole purifié, insolubilisé dans le tétrachlorure de carbone ou le tétrachloro-1,1,2,2 éthylène est séparé par tous moyens physiques connus, sans traitement alcalin préalable.

**Claims**

1. Process for the purification of mercaptobenzothiazole prepared according to known processes, characterised in that the crude product is treated in the absence of water with carbon tetrachloride or 1,1,2,2-tetrachloroethylene.

2. Process according to claim 1 in which the crude product is treated with the carbon tetrachloride or the 1,1,2,2-tetrachloroethylene without previous separation of the volatile substances.

3. Process according to claim 1 in which the product is treated with the carbon tetrachloride or the 1,1,2,2-tetrachloroethylene after previous separation of the volatile substances by distillation under reduced pressure.

4. Process according to claim 3 in which the distillation is effected at a temperature lower than 220°C under a pressure of 1333 to 26664 pascals.

5. Process according to each of claims 1—3 characterised in that the crude product is put into suspension in the carbon tetrachloride or the 1,1,2,2-tetrachloroethylene at a temperature equal to or lower than the boiling temperature of the solvent.

6. Process according to each of claims 1—5 characterised in that the purified mercaptobenzothiazole, rendered insoluble in the carbon tetrachloride or the 1,1,2,2-tetrachloroethylene is separated by all known physical means, without previous alkaline treatment.

**Patentansprüche**

1. Verfahren zur Reinigung von in an sich be-kannter Weise hergestelltem Mercaptobenzo-

thiazol, dadurch gekennzeichnet, daß man das rohe Produkt in Abwesenheit von Wasser mit Tetrachlorkohlenstoff oder 1,1,2,2-Tetrachloräthylen behandelt.

2. Verfahren nach Anspruch 1, dadurch gekennzeichnet, daß man das rohe Produkt ohne vorherige Abtrennung flüchtiger Materialien mit Tetrachlorkohlenstoff oder 1,1,2,2-Tetrachloräthylen behandelt.

3. Verfahren nach Anspruch 1, dadurch gekennzeichnet, daß man das rohe Produkt nach der vorherigen Abtrennung der flüchtigen Materialien durch Destillation unter Vermindertem Druck mit Tetrachlorkohlenstoff oder 1,1,2,2-Tetrachloräthylen behandelt.

4. Verfahren nach Anspruch 3, dadurch gekennzeichnet, daß man die Destillation bei einer Temperatur unterhalb 220°C bei einem Druck von 1333 bis 26664 Pa durchführt.

5. Verfahren nach einem der Ansprüche 1 bis 3, dadurch gekennzeichnet, daß man das rohe Produkt bei einer Temperatur, die der Siedetemperatur des Lösungsmittels entspricht oder darunter liegt, in Tetrachlorkohlenstoff oder 1,1,2,2-Tetrachloräthylen suspendiert.

6. Verfahren nach einem der Ansprüche 1 bis 5, dadurch gekennzeichnet, daß man das gereinigte und in Tetrachlorkohlenstoff oder 1,1,2,2-Tetrachloräthylen unlöslich gemachte Mercaptobenzothiazol mit Hilfe an sich bekannter physikalischer Verfahrensweisen ohne vorherige Alkalibehandlung abtrennt.